# EUROPEAN PATENT APPLICATION

(11) **EP 4 464 408 A1**
(43) Date of publication of application: **20.11.2024**
(21) Application number: 23305768.6
(22) Date of filing: 15.05.2023
(51) Int. Cl.: B01J 19/00

(54) **METHOD OF FLUID CIRCULATION AND INTERACTION IN A SETUP FOR IMPLEMENTING A MULTI-STEP CHEMICAL PROCESS**

(71) Applicant: Sanofi, 75017 Paris (FR); Centre National de la Recherche Scientifique, 75016 Paris (FR); Ecole Nationale Superieure de Chimie Paris, 75231 Paris Cedex 05 (FR)
(72) Inventor: ARVEILER, Maël, 75017 Paris (FR)
(74) Representative: Cabinet Nony

(57) **Abstract**

The invention relates to a method of fluid circulation in a setup for implementing a multi-step chemical process, comprising:
- supplying (102), through a first fluidic circulation system (10a) of the setup and a second fluidic circulation system (10b) of the setup (5) configured in a first supply setting, reagents (R1; R2) for performing a first step of the multi-step chemical process, to a fluidic interaction structure (25; 30) of the setup,
- collecting (104), in an intermediate product container (37a, 37b) of the setup, a product (R4) obtained from the interaction between the reagents within the fluidic interaction structure (25, 30) during the first step of the multi-step chemical process, and
- supplying (106), through the first fluidic circulation system (10a) and the second fluidic circulation system (10b) configured in a second supply setting different from the first supply setting, reagents for performing a second step of the multi-step chemical process, to the fluidic interaction structure (25; 30), and
wherein the reagents for performing the second step include the product from the first step collected in the intermediate product container (37a, 37b) or a product obtained from the interaction between said collected product and another reagent within another fluidic interaction structure (30).

## Description

The present invention relates to a method of fluid circulation and interaction in a setup for implementing a multi-step chemical process, a computer implemented method and a computer program product.

In the field of total organic synthesis, and especially medicinal chemistry, the ability to achieve multi-step processes is a prerequisite. Such complex processes in flow have been reported, either at the cost of high engineering cost or with specific synthetic routes requiring no action on the crude mixtures between reactions of the route. In practice, in-line flow setups for multi-step processes, especially with quenching or cleaning steps, are inherently complex to put in place and often inadequate for small-scale work, as is desired in drug discovery. Indeed, the hardware required for continuous flow chemistry increases linearly with the number of steps in the process, each step generally consisting in the addition of at least one inlet flow and the addition of a fluidic tool through which the addition of the flows from the previous step and from the new inlet passes.

To sum up, multi-step in-line flow chemical reaction systems are well known in the art, but are mostly limited to a specific type of reaction or reaction sequence in view of their complexity.

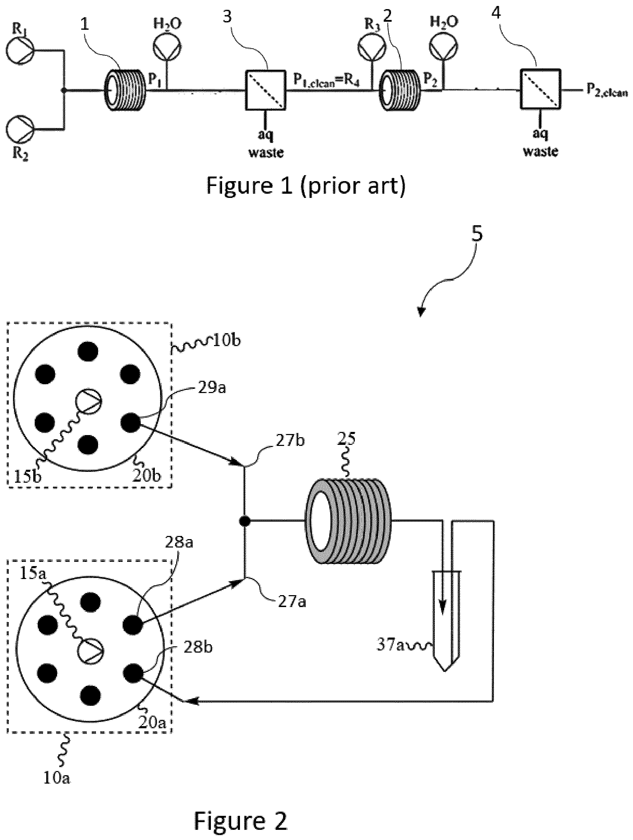

Most of these systems perform linear syntheses, where the desired target compound is constructed stepwise from a single starting material. A schematic example of a multi-step process consisting in two telescoped reactions by two reactors indicated 1 and 2 with an aqueous quench (or work-up) by two liquid-liquid separators 3 and 4 after each reaction step is presented in Figure 1. Despite the simplicity of the process, an experimental bench dedicated to it would be cluttered with five pumps, two reactors 1 and 2 and two step separators 3 and 4, to which must be added the tubes and connections between all these tools. Similarly, to the spatial footprint, the total cost of the set-up increases with the number of tools, hence with the number of steps.

Added to the previously cited drawbacks, the salient obstacle of in-line flow chemistry to multi-step processes is the dependence of downstream steps on upstream steps, especially regarding flowrates. For example, with the process presented in Figure 1, the parameters of the second reaction step are subjected to the flowrate of R4, imposed by the first reaction step with reactor 1. Consequently, the flowrate of R3 must be set accordingly to match molar fluxes as desired. Following up downstream, the second reactor 2 occurs must be chosen so as to obtain the desired residence time. The same reasoning applies iteratively to the steps downstream. An obvious but noteworthy fact is that all flowrates of miscible solvents add up, hence an increase of flowrate along the process: the total flowrate in the second reactor 2 is necessarily higher than in the first reactor 1. Thus, if the first reaction is much faster than the second one, either low flowrates must be used in the first reactor, or the second reactor must be very long, which is often not realistic. In general terms, the productivity of upstream steps is highly impacted by slow downstream steps and the setup of such multi-stem in-line flow is highly complicated.

Accordingly, it would be desirable to provide a single versatile flow method for automated multistep synthesis that is simple to design and perform, cheaper and allow to design complex multi-step processes easily.

### Summary of the invention

According to a first of its aspects, a subject of the invention is a method of fluid circulation and interaction in a setup for implementing a multi-step chemical process, comprising:
- supplying, through a first fluidic circulation system of the setup and a second fluidic circulation system of the setup configured in a first supply setting, reagents for performing a first step of the multi-step chemical process, to a fluidic interaction structure of the setup,
- collecting, in an intermediate product container of the setup, a product obtained from the interaction between the reagents within the fluidic interaction structure during the first step of the multi-step chemical process, and
- supplying, through the first fluidic circulation system and the second fluidic circulation system configured in a second supply setting different from the first supply setting, reagents for performing a second step of the multi-step chemical process, to the fluidic interaction structure of the setup, and
wherein the reagents for performing the second step include the product from the first step collected in the intermediate product container or another product obtained from the interaction between said collected product and another reagent within another fluidic interaction structure.

With such method comprising collecting the product of first step in an intermediate product container, the strategy can be a sequential flow strategy. The fluid manipulations may be performed step-by-step by picking up and dispensing fluids through the two fluidic circulation system. The fluid manipulations are performed step-by-step by picking up and dispensing fluids through the two fluidic circulation system and the one or more fluidic interaction structures. The sequential nature of the process implies that at the end of each step, all fluids are motionless, such that that upstream and downstream notions are not relevant anymore. The constraints of a step are uncoupled from the constraints of the other steps. This is a major benefit in terms of feasibility for multi-step processes: essentially, flowrates at a given step do not depend on flowrates of the previous steps. The complexity of the process is then simply the inherent complexity of each step, rid of the complexity of intertwining steps. In practice, this allows varying parameters of a step without having to change the whole process, which highly facilitates process optimisation.

In embodiments, the multi-step chemical process could be one of photochemical, electrochemical and plasma-activated transformations.

In embodiments, the method could be used for inorganic or organic synthesis.

In embodiments, the multi-step chemical process could be non-medicinal.

The method allows cleaning of the fluidic interaction structure(s) between two steps of the multi-step chemical process without manual operation. Advantageously, cross-contamination can be avoided, and the quantity of intermediate product collected can be maximized.

For example, the method may comprise supplying a fluid, notably an inert gas, by the first and/or second fluidic circulation systems to the fluidic interaction structure(s) to flush the fluid initially contained in the said fluidic interaction system(s). In another example, a fluid, notably a liquid solvent, can be supplied by the first and/or second fluidic circulation systems to the fluidic interaction structure(s) to dilute and flush residual species, hence cleaning internal parts of said fluidic interaction structure(s).

In embodiments, in the second setting, the configuration of both the first and the second fluidic circulation system are different than in the first setting. Alternatively, in the second setting, only one of the first and second fluidic circulation system may have a different configuration than in the first setting.

In embodiments, the first fluidic circulation system comprises a multi-way distribution valve configured to selectively connect fluidically two ways together. In embodiments, the first fluidic circulation system comprises a pump configured to control the circulation of reagents in the multi-way distribution valve.

Similarly, in embodiments, the second fluidic circulation system comprises a multi-way distribution valve configured to selectively connect fluidically two ways together, In embodiments, the second fluidic circulation system comprises a pump configured to control the circulation of reagents in the multi-way distribution valve. circulation of reagents in the multi-way distribution valve.

Hence, there is one pump for each of the first and the second fluidic circulation system.

In embodiments, the first and second fluidic circulation systems each comprises a multi-way distribution valve configured to selectively connect fluidically two ways together. In embodiments, the first and second fluidic circulation systems each comprises a pump configured to control the circulation of reagents in the multi-way distribution valve.

The method further comprises switching from the first supply setting to the second supply setting by connecting different ways of the multi-way distribution valve of at least one of the first and second fluidic circulation systems, better of each of the first and second fluidic circulation systems, than the ways connected in the first supply setting.

In embodiments, at least one of the first and the second fluidic circulation system comprises more than one multi-way distribution valve. Advantageously, it allows to increase the number of ways. Increasing the number of ways enable to connect a higher number of reservoirs or fluidic interaction structures. The method comprises switching from the first supply setting to the second supply setting by connecting different ways of at least one of the multi-way distribution valves of one of the first and second fluidic circulation systems, better of each of the first and second system, than the ways connected in the first supply setting. In embodiments, the first and the second fluidic circulation system each comprises only one pump controlling all the multi-way distribution valves of the corresponding fluidic circulation system.

In embodiment, the intermediate product container is directly or indirectly connected to the outlet of the fluidic interaction structure supplied in first step.

In embodiments, the method further comprises redirecting a fluid obtained at an outlet of the fluidic interaction structure towards the intermediate product container or towards another container of the setup. This redirecting may be performed by a redirection means of the setup configured to be selectively connected to the inlet of the intermediate product container or of another container. In embodiments, the redirection means is a multi-way valve and the redirecting comprise connecting fluidically together two ways to connect the outlet of the fluidic interaction structure supplied in first step towards selectively the intermediate product container or towards another container. Such redirection allows in one selectable configuration to connect indirectly the intermediate product container to the outlet of the fluidic interaction structure supplied in first step.

In embodiments, the fluidic interaction structure(s) are chosen among reaction structure, phase separation structure, or physico-chemical characterization structure.

In embodiments, the reaction structure is for instance a thermally controlled reactor or a plasma reactor or an electrochemical reactor or a photochemical reactor or a cartridge containing solid-phase reagents or catalysts.

In embodiments, the phase separation structure is for instance a liquid-liquid and/or gas-liquid separation structure.

In embodiments, the physico-chemical characterization structure is for instance a high-performance liquid chromatography structure, a gas chromatography structure, a mass spectrometry structure or a combined high performance liquid chromatography-mass spectrometry structure or a combined gas-chromatography-mass spectrometry structure, or a nuclear magnetic resonance structure or an ultraviolet/visible spectrometer structure or an infrared spectrometer structure.

In embodiments, the method comprises supplying, through the first fluidic circulation system and the second fluidic circulation system configured in another supply setting different from the first supply setting, reagents for performing another step of the multi-step chemical process, to the other fluidic interaction structure of the setup, the reagents for performing this other step comprising the product from the first step collected in the intermediate product container. In embodiments, the other fluidic interaction structure of the setup is different from the one involved in the first step. In these embodiments, the setup may comprise a plurality of intermediate container configured to be fluidically connected each to the outlet of a different fluidic interaction structure and to one of the first and second fluidic circulation systems. The method may further comprise collecting, in another intermediate product container of the setup, a product obtained from the interaction between the reagents within the other fluidic interaction structure during the other step of the multi-step chemical process. In these embodiments, the setup may comprise a plurality of redirection means each configured to be fluidically connected to the outlet of one different fluidic interaction structure and configured to be selectively connected to the inlet of the corresponding intermediate product container or of another container. In embodiments, the method further comprises redirecting a fluid obtained at an outlet of the corresponding fluidic interaction structure towards the corresponding intermediate product container or towards another container of the setup.

In embodiments, the setup comprises two fluidic interaction structures of different types among a reaction structure, a phase separation structure, or a physico-chemical characterization structure.

In embodiments, when there is a plurality of fluidic interaction structures, they are all different. Only one copy of each modular fluidic interaction structure is needed since the setup allows to execute the steps of the multi-step chemical process one after the other: a single fluidic interaction structure can be used for as many steps needed. The sequential nature of the process implies that at the end of each step, all fluids are motionless, such that that upstream and downstream notions are not relevant anymore, ergo, the constraints of a step are uncoupled from the constraints of the other steps. This is a major benefit in terms of feasibility for multi-step processes: essentially, flowrates at a given step do not depend on flowrates of the previous steps. The complexity of the process is then simply the inherent complexity of each step, rid of the complexity of intertwining steps. In practice, this allows varying parameters of a step without having to change the whole process, which highly facilitates process optimisation.

In embodiments, the fluidic interaction structures are of different types among a reaction structure, a phase separation structure, or a physico-chemical characterization structure.

In embodiments, the setup comprises a reaction structure and a phase separation structure.

The setup may further comprise a temperature controller configured to control the temperature within the fluidic interaction structure. In embodiments, the method further comprises the control of the temperature within the fluidic interaction structure during interaction of the reagents within the said fluidic interaction structure.

In embodiments, the fluidic interaction structure is a thermally controlled reactor including the temperature controller. In other embodiments, the temperature controller is a separate device, for example a proportional-integral-derivative controller (PID), configured to control the temperature inside the fluidic interaction structure, and possibly other elements of the setup.

In embodiments, the method further comprises connecting the setup to an electronic device, especially a computer, comprising a processor arrangement and performing a method of fluid circulation and interaction in the setup by commands from the processor arrangement that executes computer readable instructions generated by a computer program product to commands.

The connection to the electronic device can be a physical connection, notably with wires, or a wireless connection.

In embodiments, the method comprises, in particular for each first and second step of the multi-step chemical process, receiving commands, in particular from the electronic device, for selecting the two ways that they fluidically connect together.

In embodiments, the method comprises, in particular for each first and second step of the multi-step chemical process, receiving commands, in particular from the electronic device, for activating or deactivating either synchronously, asynchronously with a controlled delay, or independently the pumps of the first and second circulation systems as a result of the activation commands received.

In embodiments, the method comprises, in particular for each first and second step of the multi-step chemical process, receiving commands, in particular from the electronic device, for adjusting their flowrate, the volume of reagents supplied and/or the duration of supply by the pumps of the first and second circulation systems.

In embodiments, the method comprises, in particular for each first and second step of the multi-step chemical process, receiving commands, in particular from the electronic device, for controlling the temperature within the fluidic interaction structure.

In embodiments, the method comprises, in particular for each first and second step of the multi-step chemical process, receiving commands, in particular from the electronic device, and operating the fluidic interaction structure accordingly. This is the case, for instance, when the fluidic interaction structure is an electro-chemical reactor, photo-chemical reactor or a plasma-activated reactor. The operating can comprise changing a parameter of the fluidic interaction structure.

In the case where the fluidic interaction structure is an electrochemical reactor, the operation commands may comprise a voltage value or range target or an electrical current value or range target.

In the case where the fluidic interaction structure is a photo-chemical reactor, the operation commands may comprise a light intensity value or range target or a light wavelength value or range target.

In the case where the fluidic interaction structure is a plasma-activated reactor, the operation commands may comprise a voltage value or range target, or an electrical variable signal target, in particular a sinusoid, square, triangular, or any arbitrary signal predetermined by the processor.

In embodiments, the method comprises, in particular for each first and second step of the multi-step chemical process, receiving commands, in particular from the electronic device, for connecting the outlet of the fluidic interaction structure to the inlet of the corresponding intermediate product container or of the other container.

According to another of its aspects, a subject of the invention is a computer implemented method of controlling fluid circulation in a setup for implementing a multi-step chemical process, comprising steps performed according to the method of fluid circulation and interaction previously described.

In embodiments, the control of fluid circulation comprises sending commands to the setup, in particular to the first and second fluidic circulation systems and/or to the fluidic interaction structure and/or to the temperature controller and/or to the redirection means.

The commands may comprise commands to be send to:
- each of the first or second fluidic circulation systems, in particular to each of the multi-way distribution valves, for connecting fluidically together two selected ways,
- each of the first or second fluidic circulation systems, in particular to the pumps, for activating and/or deactivating the pumps either synchronously, asynchronously with a controlled delay, or independently,
- each of the first or second fluidic circulation systems, in particular to the pumps, for adjusting the flowrate of the pumps, the volume of reagents supplied and/or the duration of supply.
- the temperature controller for controlling the temperature within at least one fluidic interaction structure,
- the fluidic interaction structure(s) for operating the fluidic interaction structure(s), and/or
- the redirection means for connecting fluidically together two selected ways.

According to another of its aspects, a subject of the invention is a computer program product comprising computer readable instructions that, when executed by a processor arrangement, causes the processor arrangement to perform a method of fluid circulation and interaction as previously described.

In embodiments, the method of fluid circulation and interaction comprises sending commands to the first or second fluidic circulation systems, to the temperature controller and/or to the fluidic interaction structure(s).

According to another aspect, the disclosure relates to a computer-readable medium having stored thereon the computer program product as defined above.

### Brief description of the drawings

The invention may be better understood from reading the following detailed description of non-limiting implementation examples thereof and from examining the appended drawing, in which:
- Figure 1 is a schematic example of a multi-step process performed in continuous flow,
- Figure 2 is a schematic view of a setup according to first embodiments,
- Figure 3 is a flowchart showing steps of an exemplary method for fluid circulation and interaction according to embodiments,
- Figure 4 is a schematic view of a setup according to a second embodiment,
- Figure 5 is a schematic view of a setup according to a third embodiment,
- Figure 6 is a schematic view of a setup according to a fourth embodiment,
- Figure 7 is a schematic view of a setup according to a fifth embodiment,
- Figure 8 is a schematic view of a setup according to a sixth embodiment,
- Figure 9 is a schematic view of a setup according to a seventh embodiment,
- Figure 10 shows an exemplary multi-step chemical process performed within the exemplary setup of Figure 9, and
- Figure 11 is a schematic view of a system for implementing a multi-step chemical process according to embodiments.

### Detailed description

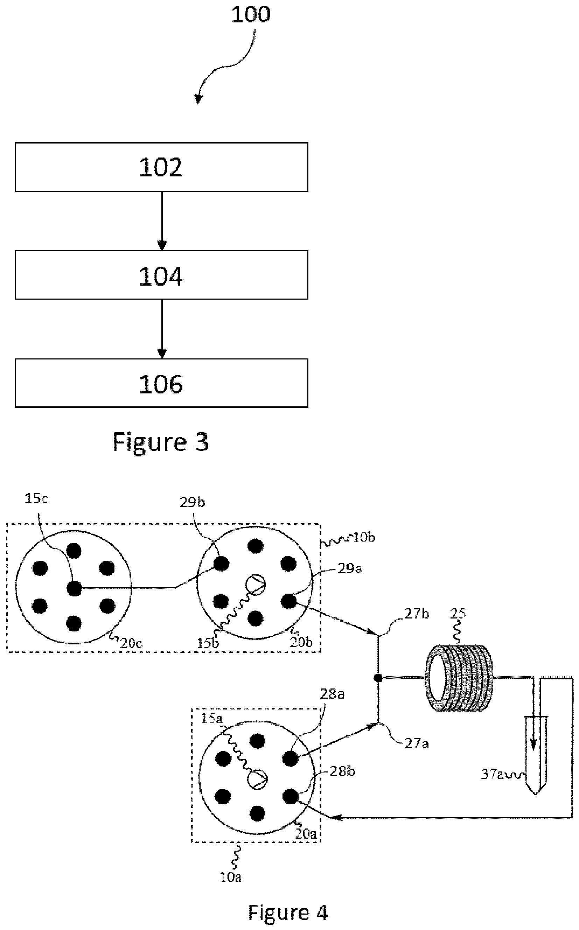

Figure 2 shows a setup 5 according to first embodiments. The setup 5 is configured to perform steps of a method of fluid circulation and interaction 100, which will be described below in reference to Figure 3.

The setup 5 comprises a first and a second fluidic circulation system 10ₐ and 10_{b}, a fluidic interaction structure 25 and an intermediate product container 37ₐ.

The fluidic interaction structure 25 comprises inlets configured to be fluidically connected to both the first and second fluidic distribution systems 10a, 10b. The first and second fluidic circulation systems 10a, 10b are configured to be in a first setting to supply the fluidic interaction structure 25 with reagents for performing a first step of the multi-step chemical process, and in a second setting, different from the first setting, to supply the fluidic interaction structure 25 with reagents for performing a second step of the multi-step chemical process.

The intermediate product container is configured to be fluidically connected to the outlet of the fluidic interaction structure 25 and to one of the fluidic circulation systems 10a, 10b. The intermediate product container 37a is configured to collect a product obtained from the interaction between the reagents within the fluidic interaction structure 25 during the first step.

In the present example, at least one of the fluidic circulation systems 10a, 10b is configured to supply the product collected in the intermediate product container 37a to the fluidic interaction structure 25 as a reagent for performing the second step.

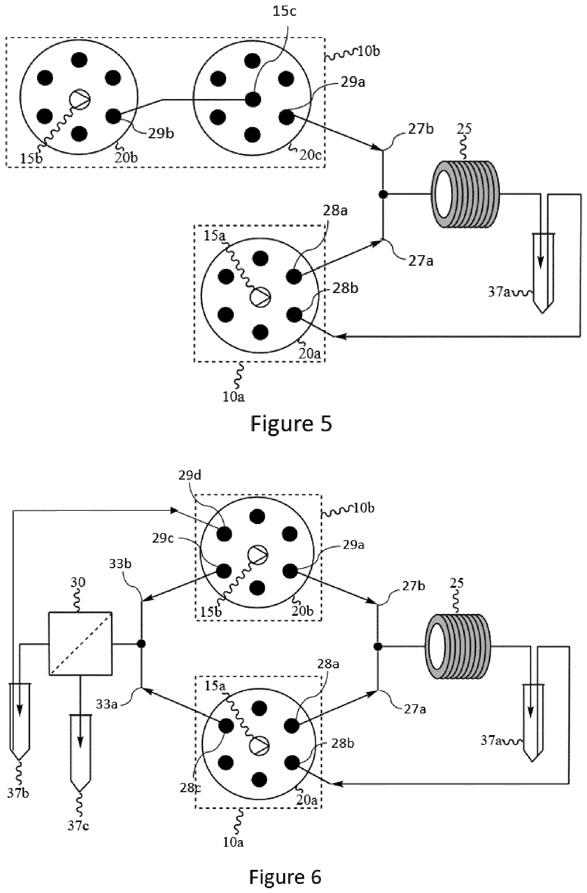

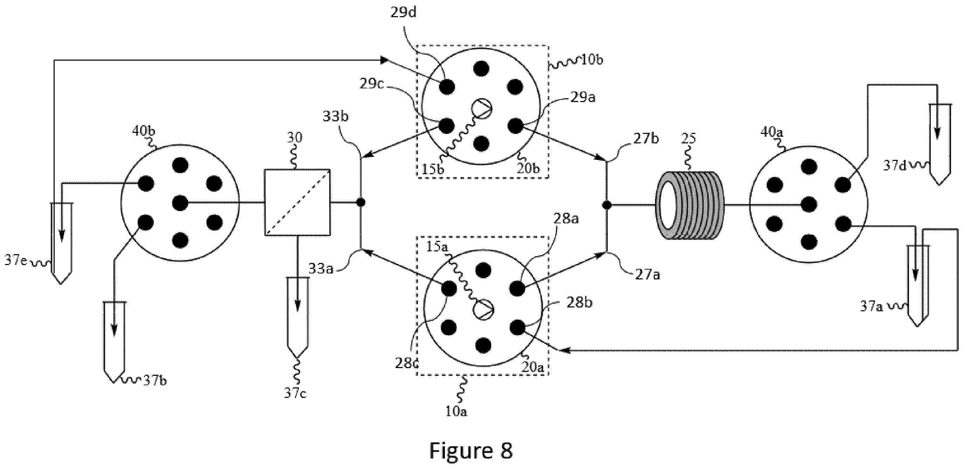

In other embodiments described below with reference to Figure 6 and 8, at least one of the fluidic circulation systems 10a, 10b is configured to supply the product collected in the intermediate product container 37a to another fluidic interaction structure 30 as a reagent for performing another step.

According to embodiments, the first and a second fluidic circulation systems 10ₐ and 10_{b} each comprises a pump 15ₐ and 15_{b} and a multi-way distribution valve 20ₐ and 20_{b}. The multi-way distribution valve 20ₐ and 20_{b} are configured to selectively connect fluidically two ways together, and the pumps 15ₐ and 15_{b} are configured to control the circulation of reagents in the multi-way distribution valves 20a, 20b. The connected ways of at least one of the multi-way distribution valves 20a, 20b are different in the first setting are different from the connected ways and in the second setting.

The fluidic interaction structure 25 is a reaction structure, a phase separation structure, or a physico-chemical characterization structure. The reaction structure is for instance a thermally controlled reactor, a plasma reactor, an electrochemical reactor, a photochemical reactor, or a cartridge containing solid-phase reagents or catalysts. The phase separation structure is for instance a liquid-liquid and/or gas-liquid separation structure. The physico-chemical characterization structure is for instance a high-performance liquid chromatography structure, a gas chromatography structure, a mass spectrometry structure or a combined high performance liquid chromatography-mass spectrometry structure or a combined gas chromatography-mass spectrometry structure, or a nuclear magnetic resonance structure or an ultraviolet/visible spectrometer structure or an infrared spectrometer structure.

In the illustrated example of Figure 2, the fluidic interaction structure 25 is a reaction structure, in particular a thermally controlled reactor. In this case, the reactor 25 comprises a temperature controller (not illustrated) configured to control the temperature within the reactor 25.

The fluidic interaction structure 25 comprises two inlets 27ₐ and 27_{b} that are connected respectively to ways 28ₐ and 29ₐ of the respective multi-way distribution valves 20ₐ and 20_{b} and an outlet connected to the intermediate product container 37ₐ.

The intermediate product container 37ₐ is further connected to a way 28_{b} of the multi-way distribution valve 20ₐ of the first fluidic circulation systems 10ₐ.

Some ways of the respective multi-way distribution valves 20ₐ and 20_{b} are connected to reagent reservoirs (not illustrated).

Figure 3 shows steps of a method 100 for fluid circulation and interaction in a setup according to embodiments. In embodiments, those steps can be performed in the setup shown in any one of Figures 2 or 4 to 9.

At a first step 102, a fluidic interaction structure 25 of the setup 5 is supplied with reagents for performing a first step of a multi-step chemical process. The supply is done through a first and a second fluidic circulation system 10a and 10b of the setup 5 that are configured in a first supply setting.

According to embodiments, the first and second fluidic circulation systems 10a, 10b each comprises a multi-way distribution valve 20a, 20b configured to selectively connect fluidically two ways together. As will be done in a later step (see step 106 below), one can switch from the first supply setting to another supply setting by connecting different ways of the multi-way distribution valves 20a and 20b than the ways connected in the first supply setting.

In some embodiments, step 102 includes receiving commands from an electronic device 500. In those embodiments, the multi-way distribution valves 20ₐ and 20_{b} may receive commands to fluidically connect the ways 28ₐ and 29ₐ to other ways connected to reservoirs comprising some reagents to be supplied for the first step of a multi-step chemical process. Upon activation of the pumps 15ₐ and 15_{b} of the respective multi-way distribution valves 20ₐ and 20_{b}, the reagents contained in the connected reservoirs are supplied to the fluidic interaction structure 25 for performing said first step. In practice, the pumps 15ₐ and 15_{b} may receive commands for activating either synchronously, asynchronously with a controlled delay, or independently. Optionally, the pumps 15ₐ and 15_{b} may also receive commands for adjusting some parameters such as their flowrate, the volume of reagents supplied and/or the duration of supply, as well as commands for deactivating. In embodiments where the setup comprises a temperature controller, it receives, at step 102, commands to set the temperature of the fluidic interaction structure 25.

At a step 104, the product obtained from the interaction between the reagents during the first step of the multi-step chemical process within the fluidic interaction structure 25 is collected in the product intermediate reservoir 37ₐ.

At a step 106, the fluidic interaction structure 25 is supplied with reagents for performing a second step of the multi-step chemical process. The supply is done through the first and the second fluidic circulation system 10a and 10b that are configured in a second supply setting different from the first supply setting. The ways that are connected fluidically together in at least one of the multi-way distribution valves 20ₐ and 20_{b} are switched to be in the second setting and allow to perform the second step. At least one of the ways connected in at least one of the multi-way distribution valves 20ₐ and 20_{b} is different in the second setting from the ways that are connected in the first setting.

In embodiments, the ways that are connected fluidically together in both multi-way distribution valves 20ₐ and 20b are switched to be in the second setting and allow to perform the second step. At least one of the ways connected in each of the multi-way distribution valves 20ₐ and 20b is different in the second setting from the ways that are connected in the first setting.

According to embodiments, the method further comprises switching from the first supply setting to the second supply setting by connecting different ways of the multi-way distribution valve than the ways connected in the first supply setting.

According to a first example, the collected product can be provided as such to the same fluidic interaction structure 25 for performance of the second step of the multi-step chemical process. Alternatively, according to a second example, the product collected in the product intermediate reservoir 37ₐ is not provided as such to the fluidic interaction structure 25 but is further transformed during an intermediary step occurring in another fluidic interaction structure 30. In this second example, the further transformed product is supplied to the fluidic interaction structure 25 as a reagent of the second step of the multi-step chemical process.

In some embodiments, step 106 includes receiving commands, in particular from an electronic device 500, as described below. The multi-way distribution valves 20ₐ and 20_{b} may receive commands to fluidically connect the ways of the multi-way distribution valve 20ₐ to a reagent reservoir comprising some reagents to be supplied for the second step of the multi-step chemical process and to the reagent of product intermediate reservoir 37ₐ comprising the collected product from the first step (in the first example) or to another product intermediate reservoir comprising the further transformed product (in the second example). Similarly to step 104, upon activation of the pumps 15ₐ and 15_{b} of the respective multi-way distribution valves 20ₐ and 20_{b}, the reagents contained in the connected reservoirs are supplied to the fluidic interaction structure 25 for performing said second step. Here again the pumps and eventually the temperature controller may receive commands to adjust some parameters.

In embodiments, the product obtained from the interaction between the reagents during the second step of the multi-step chemical process within the fluidic interaction structure 25 may be collected in the product intermediate reservoir 37ₐ (or another connected reservoir) for further use in another step of the multi-step chemical process. Advantageously, the method and the setup according to the invention are particularly useful for chemical processes comprising lots of steps.

Advantageously, the setup 5 allows cleaning of the fluidic interaction structure between two steps of the multi-step chemical process, especially without manual operation. Advantageously, cross-contamination can be avoided, and the quantity of intermediate product collected can be maximized.

For example, the first and/or second fluidic circulation systems may receive commands for supplying a fluid (in particular an inert gas) to the fluidic interaction structure to flush the fluid initially contained in the said fluidic interaction system. In another example, the first and/or second fluidic circulation systems may receive commands for supplying a fluid (in particular a liquid solvent) to the fluidic interaction structure to dilute and flush residual species, hence cleaning internal parts of said fluidic interaction structure.

Embodiments are not limited to the description above.

Figures 4 to 8 show setups according to other embodiments. Only differences with the first embodiment described with reference to Figure 2 are detailed below.

In second and third embodiments illustrated on Figures 4 and 5, the second fluidic circulation system 10_{b} comprises a pump 15_{b} and two multi-way distribution valves 20_{b} and 20_{c}. The two multi-way distribution valves 20_{b} and 20_{c} are fluidically connected, a way 29b of the one having the pump 15_{b} connected thereon being fluidically connected to the central way 15*c* of the other such that the flow imposed by the pump 15b is transmitted from one to another. The fluidic interaction structure 25 may be fluidically connected by its inlet to the one having the pump 15_{b} connected thereon, as illustrated on Figure 4, or to the other, as illustrated on Figure 5. Such fluidic circulation system with more than one multi-way distribution valves and one pump allows to easily increase the quantity of accessible ways on the fluidic circulation system that can be controlled with only one pump than with only one multi-way distribution valve. With such setups, the multi-way distribution valves 20_{b} and 20_{c} receive commands for fluidic connection of the way 29ₐ to another way connected to reagent reservoirs containing the reagents that must be inputted in the fluidic interaction structure structure 25 if necessary. Alternatively or additionally, the first fluidic circulation system 10_{b} may comprise a pump 15ₐ and two multi-way distribution valves. Alternatively, more multi-way distribution valves for each second fluidic circulation system is also possible.

In a fourth embodiment illustrated on Figure 6, the setup 5 further comprises another fluidic interaction structure 30 having inlets 33a and 33b that are connected respectively to ways 28_{c} and 29_{c} of the respective multi-way distribution valves 20ₐ and 20_{b}. In these embodiments, the fluidic interaction structure 30 is a phase separation structure. It has two outlets, one for each phase, connected each to an intermediate product container 37_{b} and 37_{c}. The container 37_{b} is connected to the multi-way valves 20_{b} through the way 29_{d}. In these embodiments, at step 106, the multi-way distribution valve 20ₐ and 20_{b} receive commands for fluidic connection of the way 28_{b} to the way 28_{c} of the multi-way distribution valve 20ₐ and the way 29_{c} to a way connected to a reagent reservoir containing a reagent. The products that are separated within the fluidic interaction structure 30 are received in the intermediate product container 37_{b} and 37_{c}. In these embodiments, the multi-way distribution valve 20ₐ and 20_{b} receive commands for fluidic connection of the way 29_{d} to the way 29ₐ of the multi-way distribution valve 20_{b} and the way 28ₐ to a way connected to a reagent reservoir containing a reagent to input the product of reservoir 37_{b} and a further reagent to the fluidic interaction structure 25.

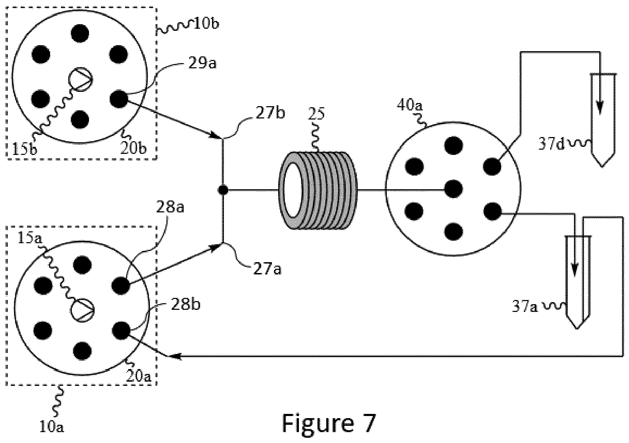

In a fifth embodiment illustrated on Figure 7, the setup 5 comprises a redirection means 40ₐ that selectively connects the outlet of the fluidic interaction structure 25 to the intermediate product container 37ₐ or to another container 37_{d}. The container 37_{d} may be or not connected to the multi-way valves 20ₐ and 20_{b}. The redirection means 40ₐ is a multi-way valve. However, it could be any means allowing redirection of the product to at least two different container and that may be commanded by a processor of a computer. At step 102, the redirection means receives commands for fluidic connection of the outlet of the fluidic interaction structure 25 to the intermediate product container 37ₐ or the other container 37_{d}.

The sixth embodiment illustrated on Figure 8 is a variant of the fifth embodiment of Figure 7, wherein the setup 5 further comprises another fluidic interaction structure 30 having inlets 33a and 33b that are connected respectively to ways 28_{c} and 29_{c} of the respective multi-way distribution valves 20ₐ and 20_{b}. In these embodiments, the fluidic interaction structure 30 is a phase separation structure. It has two outlets, one for each phase, one being connected to an intermediate product container 37_{c} and the other being connected to a redirection means 40_{b} that selectively connects the outlet of the fluidic interaction structure 30 to two containers 37_{b} and 37ₑ. The redirection means 40_{b} is a multi-way valve. However, it could be any means allowing redirection of the product to at least two different container and that may be commanded by a processor of a computer. The container 37ₑ is connected to the multi-way valves 20_{b} through the way 29_{d}. In these embodiments, at the step 106, the multi-way distribution valve 20ₐ and 20_{b} receive commands for fluidic connection of the way 28_{b} to the way 28_{c} of the multi-way distribution valve 20ₐ and the way 29_{c} to a way connected to a reagent reservoir containing a reagent. The redirection means 40_{b} receives commands for fluidically connecting the outlet of the fluidic interaction structure 30 to the intermediate product containers 37_{b} or 37ₑ. In these embodiments, the multi-way distribution valve 20ₐ and 20_{b} receive commands for fluidic connection of the way 29_{d} to the way 29ₐ of the multi-way distribution valve 20_{b} and the way 28ₐ to a way connected to a reagent reservoir containing a reagent to input the product of reservoir 37_{b} and a further reagent to the fluidic interaction structure 25.

Complex multi-step chemical processes can be performed using the set-up by taking advantage of the intermediate product containers 37a-d and of the multiplicity of ways on the multi-way distribution valves 20a and 20b. The following example is an illustration of a possible multi-step chemical process that can be performed within the setup.

### Example

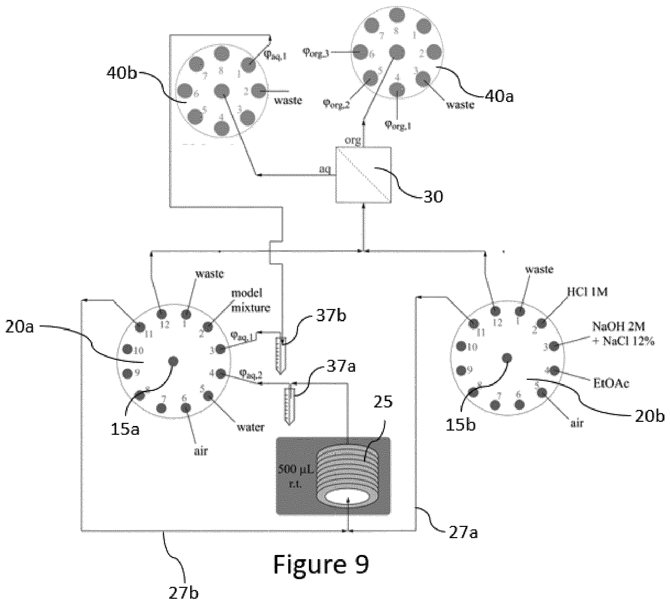

An example is presented below. The multi-step process is performed with the setup of Figure 9. The setup is based on two fluidic circulation systems 10ₐ and 10_{b} comprising each a micro-dispenser equipped with a 12-position distribution valve 20ₐ and 20_{b} and a pump 15ₐ and 15_{b}. The setup 5 further comprises a heated reactor 25 that has inlets 27ₐ and 27_{b} fluidically connected to respective ways 11 of the 12-position distribution valves 20ₐ and 20_{b} and outlet connected to an intermediate product reservoir 37ₐ. The setup 5 further comprises a phase separator 30. The phase separator 30 has inlets 33ₐ and 33_{b} fluidically connected to respective ways 12 of the 12-position distribution valves 20ₐ and 20_{b} and outlets for each phase connected to the central port of a 8-position distribution valves 40b and 40c to redirect the flow towards the adequate container. One of the ways of valve 40c is connected to an intermediate product reservoir 37b that is itself connected to a way of the valve 20a.

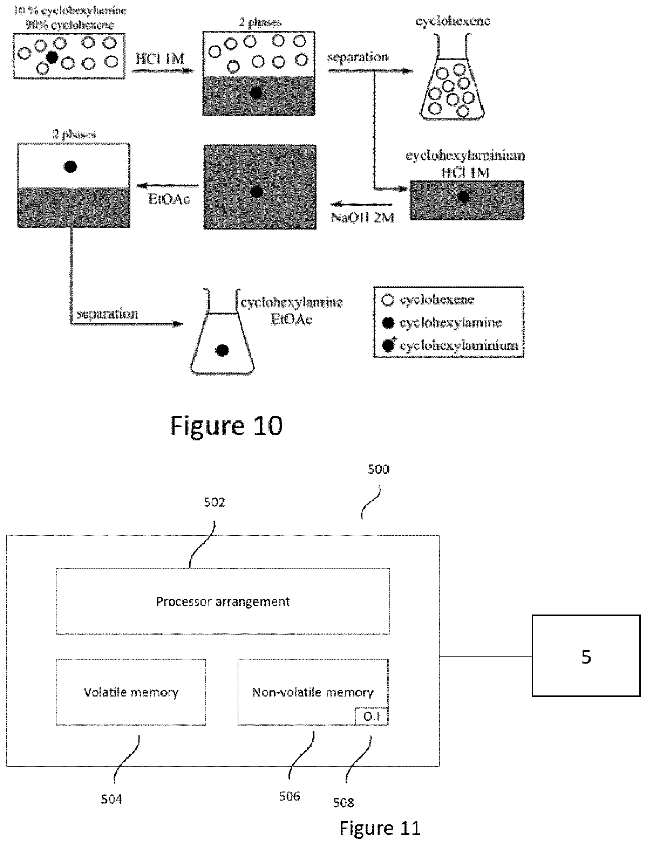

The setup is connected to a computer. The computer is configured to execute computer readable instructions generated by a computer program to command the different elements of the setup as described previously to perform a method for fluid circulation and interaction. An example of method is illustrated on Figure 10 and detailed below.

The first step consists in the liquid-liquid extraction of the amine using 1 mL of an acidic aqueous step (HCl 1 M). By separating the organic and the aqueous steps, the alkene is recovered as is, while the amine is in protonated form in the aqueous step. The ammonium ion is deprotonated using 1 mL of an alkaline brine (NaOH 2 M + NaCl 120 g/L). The amine is recovered by liquid-liquid extraction using 2 mL of an organic solvent (e.g., ethyl acetate) followed by step separation.

The method performed with the setup 5 can be summarised by the following sequence:
1. Pump 15a picks up the model mixture at way 2 of valve 20a and dispenses it to the separation structure 30 through way 12 of valve 20a while Pump 15b does the same with HCl 1 M from way 2 to way 12 of valve 20b. The organic phase resulting from the phase separation is collected in a vial through valve 40a. The aqueous phase *ϕ*_{*aq,*1} resulting from the phase separation is collected in the intermediate product container 37b through valve 40b.
2. Pump 15a picks up *ϕ*_{*aq*,1} in the intermediate product container 37b and dispenses it through the ways 3 and 11 of the valve 20a to the heated reactor 25 while Pump 15b does the same with NaOH 2 M + NaCl 120 g/L from way 3 to way 11 of valve 20b. The resulting aqueous phase *ϕ*_{*aq*,2} outputted by the heating reactor is directed to an intermediate product container 37a.
3. Pump 15a picks up *ϕ*_{*aq*,2} from the intermediate product container 37a and dispenses it through the ways 4 and 12 of the valve 20a to the extraction/separation module 30 while Pump 15b does the same from way 4 to way 12 of valve 20b with extraction solvent EtOAc. The organic phase resulting from the phase separation is collected in a vial through valve 40a. The aqueous phase resulting from the phase separation is directed to the waste through valve 40b.

Between these main events, a certain number of actions have been undertaken. The internal volumes of the tubes and of the phase separator were flushed or washed after each step by deionised water, extraction solvent and/or air to recover a maximum of the material. These intermediate operations also require movements of the pump valves and of the rotary valves.

The invention is not limited to the exemplary embodiments which have just been described. For example, the fluidic interaction structure can be different than a heated reactor or than a liquid-liquid separator and may be as mentioned above. Additional fluidic interaction structure, redirection means, container and/or intermediate reservoir container may be added.

Figure 11 shows a system for implementing a multi-step chemical process according to embodiments. The system comprises a setup 5 as previously described, which is connected physically or through a wireless connection to an electronic device 500, for instance a computer. The electronic device 500 comprises a processor configured to execute computer readable instructions of a computer program and to send commands to elements of the setup 5 to cause the setup performs steps of a method of fluid circulation and interaction 100, described in relation with Figure 3.

The electronic device 500 comprises one or more processors 502. The one or more processors control operation of other components of the electronic device 500. The one or more processors 502 may, for example, comprise a general-purpose processor. The one or more processors 502 may be a single core device or a multiple core device. The one or more processors 502 may comprise a central processing unit (CPU) and/or a graphical processing unit (GPU). Alternatively, the one or more processors 502 may comprise specialized processing hardware, for instance a RISC processor or programmable hardware with embedded firmware. Multiple processors may be included.

The electronic device comprises a working or volatile memory 504. The one or more processors may access the volatile memory 504 to process data and may control the storage of data in memory. The volatile memory 504 may comprise RAM of any type, for example Static RAM (SRAM), Dynamic RAM (DRAM), or it may comprise Flash memory, such as an SD-Card.

The electronic device comprises a non-volatile memory 506. The non-volatile memory 506 stores a set of operation instructions 508 for controlling the operation of the processors 502 in the form of computer readable instructions. The non-volatile memory 506 may be a memory of any kind such as a Read Only Memory (ROM), a Flash memory or a magnetic drive memory.

The one or more processors 502 are configured to execute operating instructions 508 to cause the setup to perform the method of fluid circulation and interaction 100. The operating instructions 508 may comprise code (i.e. drivers) relating to the hardware components of the system/apparatus 500, as well as code relating to the basic operation of the system/apparatus 500. Generally speaking, the one or more processors 502 execute one or more instructions of the operating instructions 508, which are stored permanently or semi-permanently in the non-volatile memory 506, using the volatile memory 504 to temporarily store data generated during execution of said operating instructions 508.

Implementations of the methods described below may be realized as in digital electronic circuitry, integrated circuitry, specially designed ASICs (application specific integrated circuits), computer hardware, firmware, software, and/or combinations thereof. These may include computer program products (such as software stored on e.g. magnetic discs, optical disks, memory, Programmable Logic Devices) comprising computer readable instructions that, when executed by a computer, such as that described in relation to Figure 11, cause the computer to perform one or more of the methods described herein.

Systems according to the invention may include a setup as shown in any of Figures 4 to 9.

Those of skill in the art will understand that modifications (additions and/or removals) of various components of the methods, setups, systems and embodiments described herein may be made without departing from the full scope and spirit of the present invention, which encompasses such modifications and any and all equivalents thereof.

## Claims

1. A method of fluid circulation in a setup for implementing a multi-step chemical process, comprising:
- supplying (102), through a first fluidic circulation system (10a) of the setup and a second fluidic circulation system (10b) of the setup (5) configured in a first supply setting, reagents (R1; R2) for performing a first step of the multi-step chemical process, to a fluidic interaction structure (25; 30) of the setup,
- collecting (104), in an intermediate product container (37a, 37b) of the setup, a product (R4) obtained from the interaction between the reagents within the fluidic interaction structure (25, 30) during the first step of the multi-step chemical process, and
- supplying (106), through the first fluidic circulation system (10a) and the second fluidic circulation system (10b) configured in a second supply setting different from the first supply setting, reagents for performing a second step of the multi-step chemical process, to the fluidic interaction structure (25; 30), and
wherein the reagents for performing the second step include the product from the first step collected in the intermediate product container (37a, 37b) or a product obtained from the interaction between said collected product and another reagent within another fluidic interaction structure (30).

2. The method according to claim 1, wherein the first and second fluidic circulation systems (10a, 10b) each comprises a multi-way distribution valve (20a, 20b) configured to selectively connect fluidically two ways together, and wherein the method further comprises switching from the first supply setting to the second supply setting by connecting different ways of the multi-way distribution valve (20a, 20b) of at least one of the first and second fluidic circulation systems (10a, 10b) than the ways connected in the first supply setting.

3. The method according to claim 2, comprising receiving commands for connecting fluidically together two ways of each multi-way distribution valves (20a, 20b).

4. The method according to claim 2 or 3, wherein the first and the second fluidic circulation systems (10a, 10b) each comprises a pump (15a ; 15b) configured to control the circulation of reagents in the multi-way distribution valve (20a; 20b), and wherein the method further comprises receiving commands for activating and or deactivating either synchronously, asynchronously with a controlled delay, or independently the pumps (15a, 15b), and or for adjusting the flowrate, the volume of reagents supplied and/or the duration of supply by the pumps (15a, 15b) of the first and the second fluidic circulation systems (10a, 10b).

5. The method according to any one of the preceding claims, comprising supplying, through the first fluidic circulation system (10ₐ) and the second fluidic circulation system (10_{b}) configured in another supply setting different from the first supply setting, reagents for performing another step of the multi-step chemical process, to the other fluidic interaction structure (30) of the setup, the reagents for performing this other step comprising the product from the first step collected in the intermediate product container (37ₐ, 37_{b})..

6. The method according to any one of the preceding claims, comprising controlling the temperature within the fluidic interaction structure (25, 30).

7. The method of claim 5, comprising receiving a receiving commands, for controlling the temperature within the fluidic interaction structure (25, 30).

8. The method according to any one of the preceding claims, comprising receiving commands and operating the fluidic interaction structure (25, 30).

9. The method according to any one of the preceding claims, wherein the fluidic interaction structures are chosen among a reaction structure, a phase separation structure, or a physico-chemical characterization structure.

10. The method according to any one of the preceding claims, comprising redirecting a fluid obtained at an outlet of the fluidic interaction structure (25; 30) towards the intermediate product container (37a, 37b) or towards another container of the setup.

11. A computer implemented method of controlling fluid circulation in a setup for implementing a multi-step chemical process, comprising steps according to any one of the preceding claims.

12. The computer implemented method according to the preceding claim, wherein controlling comprises sending commands to the first and second fluidic circulation systems (10a, 10b) or to the fluidic interaction structure (25, 30).

13. The computer implemented method according to the preceding claim, wherein said commands comprise commands for activating and/or deactivating pumps (15a, 15b) of the first and second fluidic circulation systems (10a, 10b) either synchronously, asynchronously with a controlled delay, or independently.

14. The computer implemented method according to the preceding claim, wherein said commands comprise commands for adjusting the flowrate of pumps (15a, 15b) of the first and second fluidic circulation systems (10a, 10b).

15. A computer program product comprising computer readable instructions that, when executed by a processor arrangement (502), causes the processor arrangement (502) to perform the computer implemented method of any one of claims 12 to 14.
